# EUROPEAN PATENT SPECIFICATION

(11) **EP 1 337 501 B1**
(45) Date of publication and mention of the grant of the patent: **20.12.2006**
(21) Application number: 01992694.8
(22) Date of filing: 22.10.2001
(51) Int. Cl.: C07C 49/67, C07C 45/67, C07C 13/465

(54) **PROCESS FOR PREPARING 1-INDANONES**
VERFAHREN ZUR HERSTELLUNG VON 1-INDANONEN
PROCEDE DE PREPARATION D' INDANONES-1

(30) Priority: 31.10.2000 EP 00203761; 21.11.2000 US 252681 P
(43) Date of publication of application: 27.08.2003
(73) Proprietor: Akzo Nobel N.V., 6824 BM Arnhem (NL)
(72) Inventor: WOUDENBERG, Richard, Herman, NL-6662 JG Elst (NL)
(74) Representative: Schalkwijk, Pieter Cornelis
(86) International application number: PCT/EP2001/012413
(87) International publication number: WO 2002/036537

(56) References cited:
- US-A- 5 840 948
- R.W. LAYER AND L.R. MAC GREGOR: "Preparation of 1-Indanones from alpha-Bromoaralkyl Ketones" JOURNAL OF ORGANIC CHEMISTRY, vol. 21, 1956, pages 1120-1123, XP002070587
- CHEMICAL ABSTRACTS, vol. 48, no. 10, 1954 Columbus, Ohio, US; Y. SHIGERU ABD U. MAKOTO: "Allied Compounds of Vitamin B1. X. Structure of N-substituted Dithiourethans" page 5866; XP002159105 & J. PHARM. SOC. JAPAN, vol. 73, 1953, pages 627-631,
- "Y. Sawaki and Y. Ogata" JOURNAL OF THE AMERICAN CHEMICAL SOCIETY, vol. 97, no. 24, 1975, pages 6983-6989, XP000973622

## Description

The present invention relates to a process for preparing 1-indanones. It further pertains to the preparation of the corresponding indenes.

1-Indanones, which can be converted into the corresponding indene derivatives by known methods, are important intermediates in the synthesis of metallocene catalysts which typically are used in combination with a co-catalyst such as methylaluminoxane for the (co)polymerization of ethylenically unsaturated monomers, e.g., the production of isotactic polypropylene.

Several processes for preparing 1-indanones which start from either a propionic acid or an acrylic acid derivative are known in the art, but none of the prior art documents describe the process of the present invention.

For example, US 5,840,948 describes a one-step process for preparing 1-indanones from benzene or a derivative thereof and a derivative of propionic acid carrying a leaving group in the α-position using a Friedel-Crafts catalyst. The starting materials in this process typically contain two halogen atoms, preferably bromine or chlorine. In all examples, a dibrominated propionic acid derivative is used.
A disadvantage of the process of US 5,840,948 is that the bromine or hydrobromic acid which results from the reaction presents a waste problem in terms of the presence and the amount of bromine-containing products.

DE 19637128 describes the reaction of an indane or tetralin derivative with a substituted acryloyl halide using a Friedel-Crafts catalyst.

A disadvantage of the process described in DE 19637128 is that the acryloyl-containing starting material is sensitive to dimerization and polymerization and that it is toxic.

R. W. Layer and I. R. MacGregor in the Journal of Organic Chemistry, Vol. 21, 1956, pp. 1120-1123, describe a process for the preparation of 1-indanones from α-bromoaralkyl ketones. It is mentioned that α-bromoaralkyl ketones are used because they are readily available. In the examples, bromine is used for preparing the α-bromoaralkyl ketones.
As described above, the use of bromine and the formation of bromine-containing products presents a waste problem.

The process according to the present invention avoids these disadvantages, presents a solution to the waste problem, and allows for the preparation of 1-indanones in high yield and selectivity.

According to the present invention, a process is provided for preparing 1-indanones of formula I: 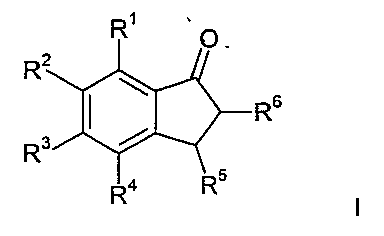 and isomers thereof, wherein R¹, R², R³, R⁴, R⁵, and R⁶ independently represent H or a C₁-C₂₀ hydrocarbon group or R¹ and R² or R² and R³ or R³ and R⁴ and/or R⁵ and R⁶ together with the carbon atoms to which they are attached form a saturated or unsaturated 5- or 6-membered ring, said hydrocarbon group and/or said ring optionally containing one or more hetero atoms, said ring optionally being substituted with a C₁-C₄ hydrocarbon group, said process comprising reacting a compound of formula II: 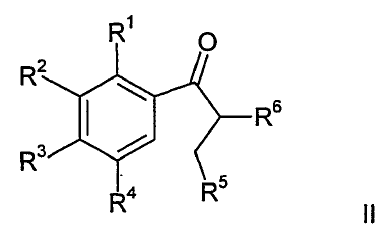 wherein R¹, R², R³, R⁴, R⁵, and R⁶ have the same meaning as defined above, with a chlorinating agent, followed by reaction with a Friedel-Crafts catalyst.

It is to be noted that the regioselectivity of the ring closure reaction with the Friedel-Crafts catalyst is dependent on the presence or absence as well as the types of R-group substituents in the compound of formula II. It may be that more than one isomer is formed during this ring closure reaction, as can be seen in Example 3 described below. Hence, the invention process relates to 1-indanones of formula I and isomers thereof.

Suitable C₁-C₂₀ hydrocarbon groups include C₁-C₂₀ alkyl, C₁-C₁₀ alkoxy, C₂-C₂₀ alkenyl, C₂-C₂₀ alkynyl, C₆-C₂₀ aryl, C₆-C₂₀ aryloxy, C₇-C₂₀ arylalkyl, and C₇-C₂₀ alkylaryl groups, which groups optionally may contain one or more hetero atoms such as O, Si, and halogen atoms. Said groups may be linear or branched.

Preferably, R¹, R², R³, R⁴, R⁵, and R⁶ independently represent H or a C₁-C₂₀ alkyl group or R¹ and R² or R² and R³ or R³ and R⁴ and/or R⁵ and R⁶ together with the carbon atoms to which they are attached form a saturated or unsaturated 5- or 6-membered ring, said ring optionally being substituted with a C₁-C₄ hydrocarbon group. More preferably, R¹, R², R³, R⁴, R⁵, and R⁶ independently represent H or a C₁-C₄ alkyl group or R¹ and R² or R² and R³ or R³ and R⁴ and/or R⁶ and R⁶ together with the carbon atoms to which they are attached form a saturated or unsaturated 5- or 6-membered ring. Even more preferably, R¹, R⁴, and R⁵ represent H, R² and R³ together with the carbon atoms to which they are attached form a saturated 5- or 6-membered ring, and R⁶ represents H or a C₁-C₄ alkyl group. Most preferably, R⁶ represents a C₁-C₄ alkyl group.
A particularly preferred C₁-C₄ alkyl group is a methyl group.
Suitable starting materials of formula II are either commercially available or can be prepared by methods known to a person skilled in this art, such as by Friedel-Crafts acylation.

In the context of the present invention, it is well-known to a person skilled in the art what is meant by the term "chlorinating agent." Chlorination of hydrocarbons is a common organic reaction and suitable chlorinating agents include chlorine, N-chlorosuccinimide, and sulfuryl chloride. For other suitable chlorinating agents the reader is referred to J. March, Advanced Organic Chemistry, Fourth Edition, John Wiley & Sons, New York, 1992, pp. 587-590. Preferred chlorinating agents are chlorine and sulfuryl chloride. In the process of the present invention most preferably sulfuryl chloride is used. Chlorine-containing salts that result from the chlorination reaction typically are discarded via the waste water. Surprisingly, the use of a chlorinating agent, in particular sulfuryl chloride, gave chlorinated products in a very high, nearly quantitative yield (see the Examples).

Typically, an acid or base catalyst in a conventional amount is used in the chlorination reaction. A practical acid catalyst is sulfuric acid, methanesulfonic acid or p-toluenesulfonic acid. A preferred chlorination catalyst is concentrated sulfuric acid.

Chlorination can be carried out in the absence or presence of a solvent. Suitable solvents include hydrocarbon solvents such as pentane, hexane, heptane, and toluene, and halogenated alkanes such as dichloromethane. Mixtures of solvents may also be used. Preferably, the reaction is carried out using a minimal amount of a solvent such as heptane.

An advantage of the present invention process is that it can be carried out at a relatively high concentration, which results in a higher reactor filling and a more economical process as compared to the processes of the prior art.

Chlorination can be carried out in a wide temperature range. Typically, it is performed at from 0°C up to 100°C, preferably at from room temperature up to 100°C. A preferred temperature range for carrying out the chlorination at pilot plant scale (typically carried out in a 1,000 litres reactor) is 50 to 70°C.

Typically, the molar ratio of ketone (II) to chlorinating agent is 1:1 to 1:2. Preferably, it is 1:1 to 1:1.5, more preferably 1:1.1 to 1:1.2. Most preferably, a molar excess of about 10% of the chlorinating agent is used. Preferably, the excess of chlorinating agent is removed from the reaction product - in a conventional way, e.g., by evaporation or via destruction with water - before further reaction.

Typical reaction times for the chlorination reaction are in the order of 15 minutes to 4 hours.

In the invention process, the chlorination reaction is followed by a ring closure reaction using a Friedel-Crafts catalyst.

Suitable Friedel-Crafts catalysts are known in the art and are described, for example, in J. March, Advanced Organic Chemistry, Fourth Edition, pp. 535-542. Typically, these catalysts are Lewis acid catalysts. Examples of suitable catalysts include aluminium chloride and iron (III) chloride. A preferred catalyst is aluminium chloride.

Typically, the ring closure reaction is carried out in the presence of a conventional solvent. Suitable solvents are those which have been described above for the chlorination reaction - with the exception of toluene - and the same solvent or mixture of solvents may be used for the ring closure reaction. Preferably, a solvent comprising a halogenated alkane such as dichloromethane is used for the ring closure reaction. Mixtures of a hydrocarbon solvent such as heptane and a halogenated solvent such as dichloromethane are particularly suitable for carrying out the ring closure reaction. Preferably, the ring closure reaction is carried out in a mixture of the hydrocarbon solvent which has been used for the chlorination reaction, such as heptane, and a halogenated solvent such as dichloromethane.

The ring closure reaction can be carried out in a wide temperature range. Typically, it is performed at from 0°C up to 100°C, in particular at from room temperature up to 100°C. A practical temperature for carrying out the ring closure reaction at laboratory scale as well as at pilot plant scale is room temperature.

Typically, the molar ratio of Friedel-Crafts catalyst used in the invention process to ketone (II) is 1:1 to 3:1. Preferably, it is 1:1 to 1.5:1, more preferably 1.2:1 to 1.3:1.

A typical reaction time for the ring closure reaction is 15 minutes to 2 hours, although longer reaction times were observed in some experiments.

The invention process can be carried out using means and equipment well-known to a person skilled in this art.

The invention process is particularly suitable for preparing 2-substituted 1-indanones. Examples have been described below.

As is known to a person skilled in the art, the 1-indanones of formula I may be reduced to the corresponding indenes in an inert solvent in two reaction steps using a reducing agent and an acid dehydrating agent. Thus, the present invention also pertains to a process for preparing indenes of formula III: 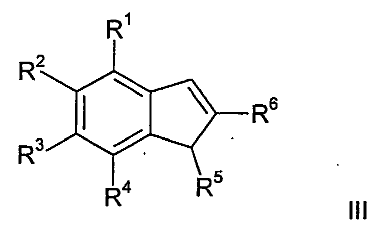 and isomers thereof, wherein R¹-R⁶ have the meaning described above and which process is characterized in that a compound of formula II is converted into a compound of formula I according to the process described above, followed by reaction in an inert solvent first with a reducing agent and then with an acid dehydrating agent according to methods known *per se.*

Suitable reducing agents include sodium borohydride, lithium aluminium hydride, diisobutylaluminium hydride (DIBAL-H), and sodium bis(2-methoxyethoxy)aluminium hydride. For these and other suitable reducing agents the reader is referred to J. March, Advanced Organic Chemistry, Fourth Edition, John Wiley & Sons, New York, 1992, pp. 910-918. A particularly preferred reducing agent is DIBAL-H.
Suitable acids include sulfuric acid, methanesulfonic acid, p-toluenesulfonic acid, and phosphoric acid. For these and other suitable acids the reader is referred to J. March, Advanced Organic Chemistry, Fourth Edition, John Wiley & Sons, New York, 1992, pp. 1011-1012. A particularly preferred acid is p-toluenesulfonic acid.
Suitable solvents include alkanes such as pentane, hexane, heptane, toluene, and xylene, halogenated alkanes such as dichloromethane and chloroform, and ethers such as tetrahydrofuran.

The present invention is illustrated by the following Examples.

### Example 1. Synthesis of 2-methylindene

### Preparation of 2-chloro-2-methylpropiophenone:

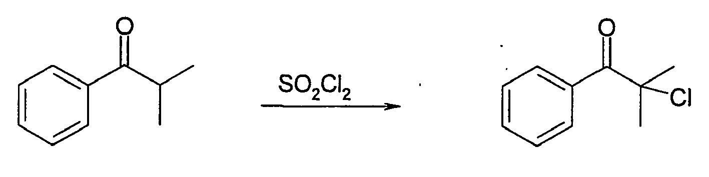

Isobutyrophenone (30.0 g, 0.20 mole) was dissolved in heptane (10 ml) and sulfuryl chloride (35 g, 0.26 mole) was added at once. After a few minutes gas evolution occurred. After 3 h of stirring at room temperature the reaction mixture was concentrated. The concentrate was dissolved in heptane (250 ml) and washed with 150 ml of an aqueous 2 wt% sodium bicarbonate solution. After drying over magnesium sulfate the solvent was removed under reduced pressure. The yield of 2-chloro-2-methylpropiophenone was 36.4 g or 98%.
¹H-NMR spectrum (400 MHz, CDCl₃): 1.86 (s, 6H), 7.42 (t, 2H), 7.53 (t, 1H),8.13 (d, 2H).

### Preparation of 2-methyl-1-indanone:

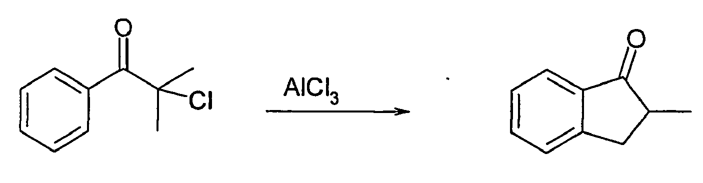

Aluminium chloride (45 g, 0.34 mole) was suspended in 200 ml of anhydrous dichloromethane. 2-Chloro-2-methylpropiophenone (30.0 g, 0.16 mole) in 30 ml of heptane was slowly added to this suspension. During the addition (30 min) the temperature was kept at room temperature. After the addition was completed, the reaction mixture was stirred overnight at room temperature.
The reaction mixture was poured onto 400 g of ice water, and the organic layer was separated. The organic layer was successively washed twice with an aqueous 5 wt% hydrochloric acid solution (100 ml) and once with an aqueous 2 wt% sodium bicarbonate solution (150 ml). After drying over anhydrous magnesium sulfate the solution was concentrated under reduced pressure. The yield of crude 2-methyl-1-indanone was 22.6 g or 94%. Distillation of the crude material gave 2-methyl-1-indanone as a light yellow liquid.
¹H-NMR spectrum (400 MHz, CDCl₃): 1.31 (d, 3H), 2.73 (m, 2H), 3.40 (dd, 1 H), 7.36 (t, 1 H), 7.45 (d, 1 H), 7.58 (t, 1 H), 7.75(d, 1 H).

### Preparation of 2-methylindene:

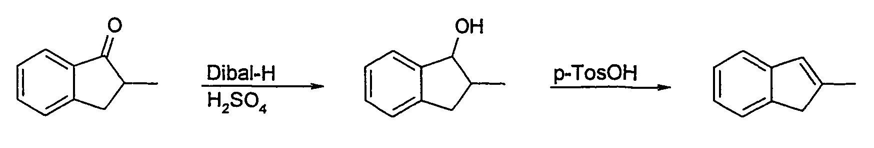

Diisobutylaluminium hydride (6 g, 42 mmoles) was added dropwise to 2-methyl-1-indanone (5.0 g, 34 mmoles) dissolved in 50 ml of anhydrous toluene, while the reaction temperature was kept at room temperature. The reaction mixture was stirred for 0.5 h after the addition. The reaction mixture was hydrolyzed by being added dropwise to sulfuric acid (aqueous 25 wt%, 50 ml) at 70°C. After the addition the aqueous layer was discarded, and the organic layer was washed twice with sulfuric acid (aqueous 5 wt%, 50 ml) and once with water (50 ml).

p-Toluenesulfonic acid (50 mg) was added to the organic layer, and the reaction mixture was heated under reflux with water separation for 1 h. After cooling to room temperature the organic layer was washed with sodium bicarbonate (aqueous 2 wt%, 50 ml), dried over magnesium sulfate, and carefully concentrated under reduced pressure. The yield of 2-methylindene was 4.2 g or 94%.
¹H-NMR spectrum (400 MHz, CDCl₃): 2.13 (s, 3H), 3.27 (s, 2H), 6.47 (s, 1H), 7.08 (d, 1 H), 7.09-7.17 (m, 2H), 7.33 (d, 1 H).

### Example 2. Synthesis of 2,4,6-trimethylindene

### Preparation of 2,2',4'-trimethylpropiophenone:

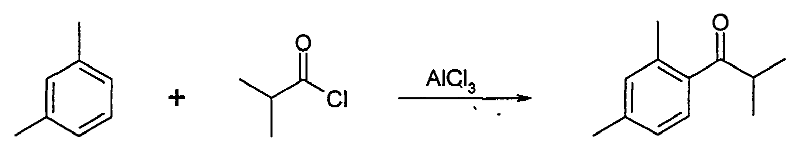

Aluminium chloride (28.0 g, 0.21 mole) was suspended in 200 ml of anhydrous dichloromethane and cooled to 0°C. A solution of m-xylene (21.2 g, 0.2 mole) and isobutyryl chloride (21.3 g, 0.20 mole) in 50 ml of anhydrous dichloromethane was slowly added to this suspension. After the addition was completed, the reaction mixture was stirred at 0°C for 1 h.
The reaction mixture was poured onto 300 g of ice water, and the organic layer was separated. The organic layer was successively washed twice with a 5 wt% aqueous hydrochloric acid solution (150 ml) and once with a 2 wt% aqueous sodium bicarbonate solution (150 ml). After drying over anhydrous magnesium sulfate the solution was concentrated under reduced pressure. The yield of 2,2',4'-trimethylpropiophenone was 35.0 g or 99%.
¹H-NMR spectrum (400 MHz, CDCl₃): 1.17 (d, 6H), 2.35 (s, 3H), 2.41 (s, 3H), 3.37 (m, 1H), 7.03 (d, 1H), 7.04 (s, 1H),7.48 (d, 1 H).

### Preparation of 2-chloro-2,2',4'-trimethylpropiophenone:

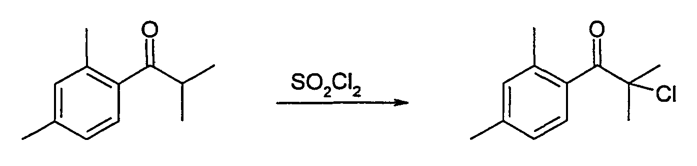

2,2',4'-Trimethylpropiophenone (30.0 g, 0.17 mole) was dissolved in heptane (10 ml), and sulfuryl chloride (35 g, 0.26 mole) was added at once. After about 5 min gas evolution occurred. After 3 h of stirring at room temperature the reaction mixture was concentrated. The concentrate was dissolved in heptane (200 ml) and was washed with 150 ml of a 2 wt% aqueous sodium bicarbonate solution. After drying over magnesium sulfate the solvent was removed under reduced pressure. The yield of 2-chloro-2,2',4'-trimethylpropiophenone was 35.5 g or 99%.
¹H-NMR spectrum (400 MHz, CDCl₃): 1.81 (s, 6H), 2.27 (s, 3H), 2.34 (s, 3H), 7.00 (d, 1 H), 7.06 (s, 1H),7.49 (d, 1H).

### Preparation of 2,5,7-trimethyl-1-indanone:

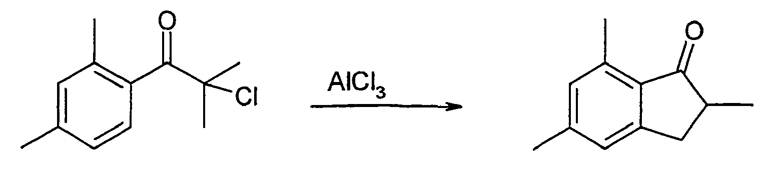

Aluminium chloride (38 g, 0.29 mole) was suspended in 200 ml of anhydrous dichloromethane. 2-Chloro-2,2',4'-trimethylpropiophenone (30.0 g, 0.14 mole) in 30 ml of heptane was slowly added to this suspension. During the addition (30 min) the temperature was kept at room temperature. After the addition was completed, the reaction mixture was stirred for 1 h at room temperature. The reaction mixture was poured onto 400 g of ice water, and the organic layer was separated. The organic layer was successively washed twice with a 5 wt% aqueous hydrochloric acid solution (150 ml) and once with a 2 wt% aqueous sodium bicarbonate solution (150 ml). After drying over anhydrous magnesium sulfate the solution was concentrated under reduced pressure. The yield of crude 2,5,7-trimethyl-1-indanone was 22.9 g or 92%. A sample was crystallized from pentane to give crystalline material.
¹H-NMR spectrum (400 MHz, CDCl₃): 1.27 (d, 3H), 2.36 (s, 3H), 2.58 (s, 3H), 3.25 (dd, 1 H), 6.90 (s, 1 H), 7.04 (s, 1 H).

### Preparation of 2,4,6-trimethylindene:

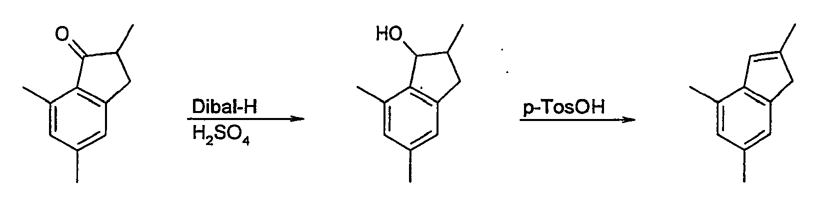

Diisobutylaluminium hydride (4.5 g, 32 mmoles) was added dropwise to a solution of 2,5,7-trimethyl-1-indanone (5.0 g, 29 mmoles) in 50 ml of anhydrous toluene, while the reaction temperature was kept at room temperature. The reaction mixture was stirred for 0.5 h after the addition. The reaction product was hydrolyzed by the reaction mixture being added dropwise to sulfuric acid (aqueous 25 wt%, 50 ml) at 70°C. After the addition the aqueous layer was discarded, and the organic layer was washed twice with sulfuric acid (aqueous 5 wt%, 50 ml) and once with water (50 ml).

p-Toluenesulfonic acid (50 mg) was added to the organic layer, and the reaction mixture was heated under reflux with water separation for 1 h. After cooling to room temperature the organic layer was washed with sodium bicarbonate (aqueous 2 wt%, 50 ml), dried over magnesium sulfate, and concentrated under reduced pressure. The yield of 2,4,6-trimethylindene was 4.4 g or 97%.
¹H-NMR spectrum (400 MHz, CDCl₃): 2.12 (s, 3H), 2.31 (s, 3H), 2.33 (s, 3H), 3.23 (s, 3H), 6.52 (s, 1 H), 6.83 (s, 1 H), 7.01 (s, 1 H).

### Example 3. Synthesis of a mixture of tetrahydro-2-methylbenzindenes

### Preparation of 2-methyl-1-(5,6,7,8-tetrahydro-2-naphthalenyl)-1-propanone:

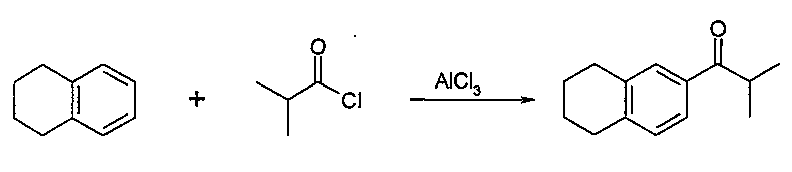

Aluminium chloride (28.0 g, 0.21 mole) was suspended in 200 ml of anhydrous dichloromethane (200 ml) and cooled to 0°C. A solution of 1,2,3,4-tetrahydronaphthalene (26.5 g, 0.2 mole) and isobutyryl chloride (21.3 g, 0.20 mole) in 50 ml of anhydrous dichloromethane was slowly added to this suspension. After the addition was completed, the reaction mixture was stirred at 0°C for 1 h. The reaction mixture was poured onto 300 g of ice water, and the organic layer was separated. The organic layer was successively washed twice with a 5 wt% aqueous hydrochloric acid solution (150 ml) and once with a 2 wt% aqueous sodium bicarbonate solution (150 ml). After drying over anhydrous magnesium sulfate the solution was concentrated under reduced pressure. The yield of 2-methyl-1-(5,6,7,8-tetrahydro-2-naphthalenyl)-1-propanone was 39.7 g or 98%.
¹H-NMR spectrum (400 MHz, CDCl₃): 1.17 (d, 6H), 1.80 (m, 4H), 2.80 (m, 4H), 3.53 (m, 1 H), 7.12 (d, 1 H), 7.66 (m, 2H).

### Preparation of 2-chloro-2-methyl-1-(5,6,7,8-tetrahydro-2-naphthalenyl)-1-propanone:

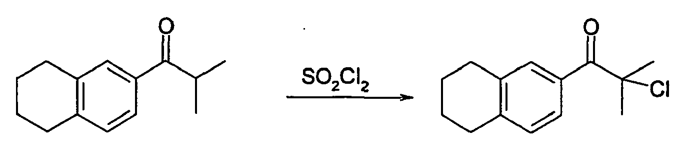

2-Methyl-1-(5,6,7,8-tetrahydro-2-naphthalenyl)-1-propanone (30.0 g, 0.15 mole) was dissolved in heptane (10 ml), and sulfuryl chloride (35 g, 0.26 mole) was added at once. After about 5 min gas evolution occurred. After 3 h of stirring at room temperature the reaction mixture was concentrated. The concentrate was dissolved in heptane (200 ml) and was washed with 150 ml of a 2 wt% aqueous sodium bicarbonate solution. After drying over magnesium sulfate the solvent was removed under reduced pressure. The yield of 2-chloro-2-methyl-1-(5,6,7,8-tetrahydro-2-naphthalenyl)-,1-propanone was 34.5 g or 98%.
¹H NMR spectrum (400 MHz, CDCl₃): 1.80 (m, 4H), 1.87 (s, 6H), 2.81 (m, 4H), 7.06 (d, 1 H), 7.84 (s, 1 H), 7.90 (d, 1 H).

### Preparation of a mixture of 2,3,6,7,8,9-hexahydro-2-methyl-1H-benz[e]inden-1-one, 2,3,5,6,7,8-hexahydro-2-methyl-1H-benz[f]inden-1-one, and 1,2,6,7, 8,9-hexahydro-2-methyl-3H-benz[e]inden-1-one:

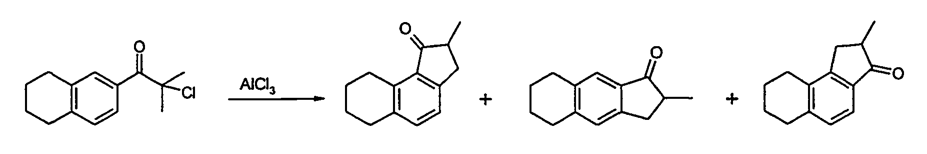

Aluminium chloride (35 g, 0.26 mole) was suspended in 200 ml of anhydrous dichloromethane. 2-Chloro-2-methyl-1-(5,6,7,8-tetrahydro-2-naphthalenyl)-1-propanone (30.0 g, 0.13 mole) in 30 ml of heptane was slowly added to this suspension. During the addition (30 min) the temperature was kept at room temperature. After the addition was completed, the reaction mixture was stirred for 1 h at room temperature. The reaction mixture was poured onto 400 g of ice water, and the organic layer was separated. The organic layer was successively washed twice with a 5 wt% aqueous hydrochloric acid solution (150 ml) and once with a 2 wt% aqueous sodium bicarbonate solution (150 ml). After drying over anhydrous magnesium sulfate the solution was concentrated under reduced pressure. The yield of the mixture of crude benzinden-1-ones was 24.5 g or 97%.
From the ¹H-NMR spectrum, the molar ratio of the isomers was determined:
2,3,6,7,8,9-hexahydro-2-methyl-1H-benz[e]inden-1-one: 70%
2,3,5,6,7,8-hexahydro-2-methyl-1H-benz[f]inden-1-one: 24%
1,2,6,7,8,9-hexahydro-2-methyl-3H-benz[e]inden-1-one: 6%

A sample was crystallized from pentane to give crystalline 2,3,6,7,8,9-hexahydro-2-methyl-1 H-benz[e]inden-1-one having a GC purity of 99.1 area%.
¹H-NMR spectrum (400 MHz, CDCl₃): 1.29 (d, 3H), 1.85 (m, 4H), 2.50 (dd, 1 H), 2.68 (m, 3H), 2.80 (m, 2H), 3.20 (dd, 1 H), 7.08 (d, 1 H), 7.48 (d, 1H).
The crystallization filtrate was concentrated and analyzed by ¹H NMR. The concentrate consisted mainly of a mixture of the two main isomers: the linear 2,3,5,6,7,8-hexahydro-2-methyl-1H-benz[f]inden-1-one and 2,3,6,7,8,9-hexahydro-2-methyl-1 H-benz[e]inden-1-one. The ¹H-NMR of the linear isomer was determined from the spectrum of the mixture of isomers.
¹H-NMR spectrum of 2,3,5,6,7,8-hexahydro-2-methyl-1H-benz[f]inden-1-one (400 MHz, CDCl₃): 1.28 (d, 3H), 1.85 (m, 4H), 2.68 (m, 4H), 2.80 (m, 2H), 3.31 (dd, 1 H), 7.13 (s, 1H), 7.46 (s, 1 H).

### Preparation of 6,7,8,9-tetrahydro-2-methyl-3H-benz[e]indene:

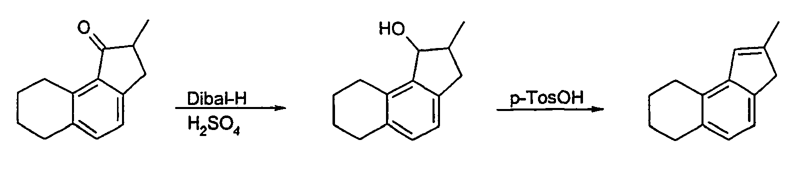

Diisobutylaluminium hydride (4.0 g, 28 mmoles) was added dropwise to a solution of 2,3,6,7,8,9-hexahydro-2-methyl-1H-benz[e]inden-1-one (5.0 g, 25 mmoles) in 50 ml of anhydrous toluene, while the reaction temperature was kept at room temperature. The reaction mixture was stirred for 0.5 h after the addition. The reaction product was hydrolyzed by sulfuric acid (aqueous 25 wt%, 50 ml) being added dropwise at 70°C. After the addition the aqueous layer was discarded, and the organic layer was washed twice with sulfuric acid (aqueous 5 wt%, 50 ml) and once with water (50 ml).

p-Toluenesulfonic acid (50 mg) was added to the organic layer, and the reaction mixture was heated under reflux with water separation for 1 h. After cooling to room temperature the organic layer was washed with sodium bicarbonate (aqueous 2 wt%, 50 ml), dried over magnesium sulfate, and concentrated under reduced pressure. The yield of crystalline 6,7,8,9-tetrahydro-2-methyl-3H-benz[e]indehe was 4.4 g or 96%.
¹H NMR spectrum (400 MHz, CDCl₃): 1.79 (m, 4H), 2.11 (s, 3H), 2.66 (t, 2H), 2.77 (t, 2H), 3.05 (s, 2H), 6.40 (s, 1 H), 6.92 (d, 1 H), 7.00 (d, 1 H).

### Preparation of a mixture of 5,6,7,8-tetrahydro-2-methyl-1H-benz[f]indene and 6,7,8,9-tetrahydro-2-methyl-3H-benz[e]indene:

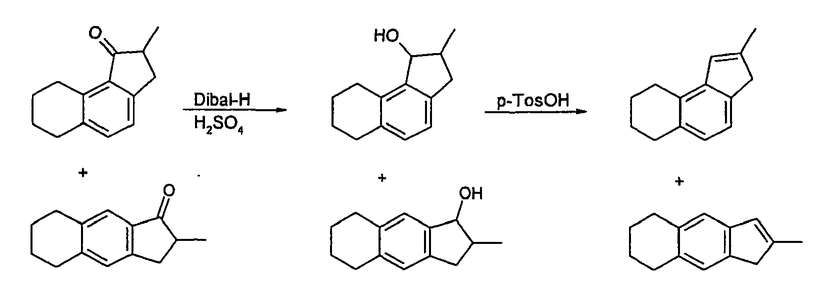

Diisobutylaluminium hydride (3.9 g, 27 mmoles) was added dropwise to a solution of a mixture of 2,3,5,6,7,8-hexahydro-2-methyl-1H-benz[f]inden-1-one and 2,3,6,7,8,9-hexahydro-2-methyl-1H-benz[e]inden-1-one (5.0 g, 25 mmoles) in 50 ml of anhydrous toluene, while the reaction temperature was kept at room temperature. The reaction mixture was stirred for 0.5 h after the addition. The reaction product was hydrolyzed by the reaction mixture being added dropwise to sulfuric acid (aqueous 25 wt%, 50 ml) at 70°C. After the addition the aqueous layer was discarded, and the organic layer was washed twice with sulfuric acid (aqueous 5 wt%, 50 ml) and once with water (50 ml).

p-Toluenesulfonic acid (50 mg) was added to the organic layer, and the reaction mixture was heated under reflux with water separation for 1 h. After cooling to room temperature, the organic layer was washed with sodium bicarbonate (aqueous 2 wt%, 50 ml), dried over magnesium sulfate, and concentrated under reduced pressure. The yield of 5,6,7,8-tetrahydro-2-methyl-1H-benz[f]indene and 6,7,8,9-tetrahydro-2-methyl-3H-benz[e]indene was 4.3 g or 93%.
The ¹H-NMR of the linear isomer was determined from the spectrum of the mixture of isomers.
¹H-NMR spectrum of 5,6,7,8-tetrahydro-2-methyl-1H-benz[f]indene (400 MHz, CDCl₃): 1.79 (m, 4H), 2.10 (s, 3H), 2.75 (m, 4H), 3.21 (s, 2H), 6.38 (s, 1 H), 6.93 (s, 1 H), 7.05 (s, 1 H).

### Example 4. Synthesis of 2-methyl-3H-benz[e]indene

### Preparation of 2-methyl-1-(1-naphthalenyl)-1-propanone:

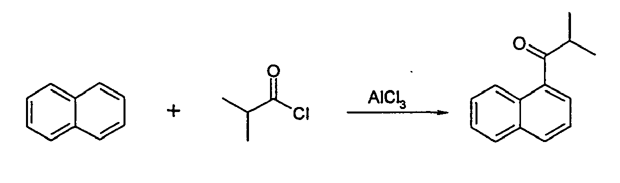

Aluminium chloride (28.0 g, 0.21 mole) was suspended in 200 ml of anhydrous dichloromethane and was cooled to -30°C. A solution of naphthalene (25.6 g, 0.2 mole) and isobutyryl chloride (21.3 g, 0.20 mole) in 50 ml of anhydrous dichloromethane was slowly added to this suspension. After the addition was completed, the reaction mixture was stirred at -30°C for 1 h. The reaction mixture was then allowed to warm up to room temperature. The reaction mixture was poured onto 300 g of ice water, and the organic layer was separated. The organic layer was successively washed twice with a 5 wt% aqueous hydrochloric acid solution (150 ml) and once with a 2 wt% aqueous sodium bicarbonate solution (150 ml). After drying over anhydrous magnesium sulfate the solution was concentrated under reduced pressure. The yield of 2-methyl-1-(1-naphthalenyl)-1-propanone was 39.7 g or 98%.
¹H-NMR spectrum (400 MHz, CDCl₃): 1.25 (d, 6H), 3.51 (m, 1 H), 7.46-7.60 (m, 3H), 7.73 (d, 1 H), 7.87 (d, 1 H), 7.95 (d, 1 H), 8.33 (d, 1 H).

### Preparation of 2-chloro-2-methyl-1-(1-naphthalenyl)-1-propanone:

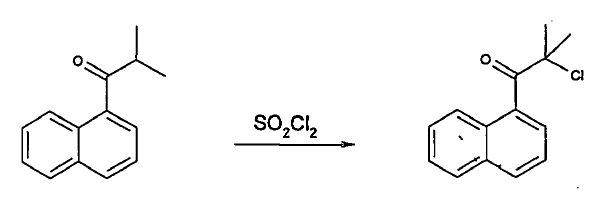

2-Methyl-1-(1-naphthalenyl)-1-propanone (30.0 g, 0.15 mole) was dissolved in heptane (10 ml), and sulfuryl chloride (30 g, 0.22 mole) was added at once. After about 5 min gas evolution occurred. After 3 h of stirring at room temperature the reaction mixture was concentrated. The concentrate was dissolved in heptane (200 ml) and washed with 150 ml of a 2 wt% aqueous sodium bicarbonate solution. After drying over magnesium sulfate the solvent was removed under reduced pressure. The yield of 2-chloro-2-methyl-1-(1-naphthalenyl)-1-propanone was 34.5 g or 98%.
¹H-NMR spectrum (400 MHz, CDCl₃): 1.89 (s, 6H), 7.42-7.58 (m, 3H), 7.74 (m, 1H), 7.79 (d, 1 H), 7.88 (m, 1 H), 7.92 (d, 1 H).

### Preparation of 2,3-dihydro-2-methyl-1H-benz[e]inden-1-one:

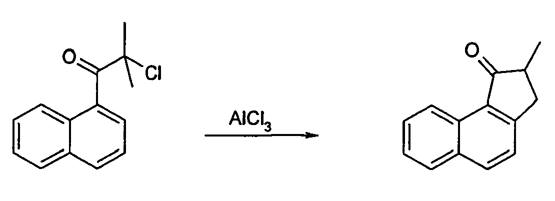

Aluminium chloride (35 g, 0.26 mole) was suspended in 200 ml of anhydrous dichloromethane. 2-Chloro-2-methyl-1-(1-naphthalenyl)-1-propanone (30.0 g, 0.13 mole) in 30 ml of heptane was slowly added to this suspension. During the addition (30 min) the temperature was kept at room temperature. After the addition was completed, the reaction mixture was stirred for 1 h at room temperature. The reaction mixture was poured onto 400 g of ice water, and the organic layer was separated. The organic layer was successively washed twice with a 5 wt% aqueous hydrochloric acid solution (150 ml) and once with a 2 wt% aqueous sodium bicarbonate solution (150 ml). After drying over anhydrous magnesium sulfate the solution was concentrated under reduced pressure. The yield of crude 2,3-dihydro-2-methyl-1H-benz[e]inden-1-one was 23.4 g or 93%. Distillation of the crude product under reduced pressure (b.p. 130°C, 0.5 mbar) gave 18.7 g (75%) of 2,3-dihydro-2-methyl-1H-benz[e]inden-1-one, which crystallized upon standing at room temperature.
¹H-NMR spectrum (400 MHz, CDCl₃): 1.37 (d, 3H), 2.80 (m, 2H), 3.45 (dd, 1 H), 7.46 (d, 1 H), 7.54 (t, 1 H), 7.65 (t, 1 H), 7.86 (d, 1 H), 8.01 (d, 1 H), 9.16 (d, 1 H).

### Preparation of 2-methyl-3H-benz[e]indene:

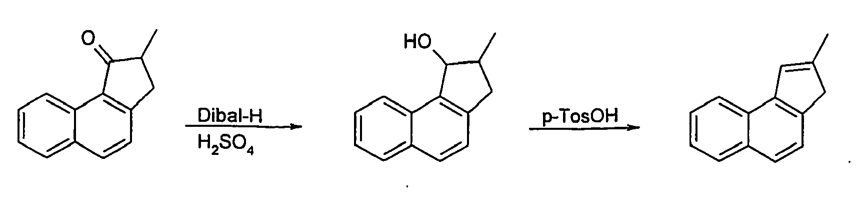

Diisobutylaluminium hydride (4.0 g, 28 mmoles) was added dropwise to a solution of 2,3-dihydro-2-methyl-1H-benz[e]inden-1-one (5.0 g, 26 mmoles) in 50 ml of anhydrous toluene, while the reaction temperature was kept at room temperature. The reaction mixture was stirred for 0.5 h after the addition. The reaction product was hydrolyzed by the reaction mixture being added dropwise to sulfuric acid (aqueous 25 wt%, 50 ml) at 70°C. After the addition the aqueous layer was discarded, and the organic layer was washed twice with sulfuric acid (aqueous 5 wt%, 50 ml) and once with water (50 ml).

p-Toluenesulfonic acid (50 mg) was added to the organic layer, and the reaction mixture was heated under reflux with water separation for 1 h. After cooling to room temperature the organic layer was washed with sodium bicarbonate (aqueous 2 wt%, 50 ml), dried over magnesium sulfate, and concentrated under reduced pressure. The yield of 2-methyl-3H-benz[e]indene was 4.1 g or 89%.
¹H-NMR spectrum (400 MHz, CDCl₃): 2.25 (s, 3H), 3.42 (s, 2H), 7.06 (s, 1H), 7.40 (t, 1 H), 7.45 (t, 1 H), 7.52 (d, 1 H), 7.59 (d, 1 H), 7.84 (d, 1 H), 8.02 (d, 1 H).

## Claims

1. A process for preparing 1-indanones of formula I: 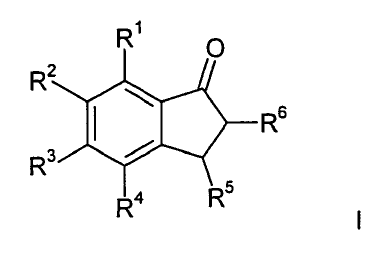 and isomers thereof, wherein R¹, R², R³, R⁴, R⁵, and R⁶ independently represent H or a C₁-C₂₀ hydrocarbon group or R¹ and R² or R² and R³ or R³ and R⁴ and/or R⁵ and R⁶ together with the carbon atoms to which they are attached form a saturated or unsaturated 5- or 6-membered ring, said hydrocarbon group and/or said ring optionally containing one or more hetero atoms, said ring optionally being substituted with a C₁-C₄ hydrocarbon group,
said process comprising reacting a compound of formula II: 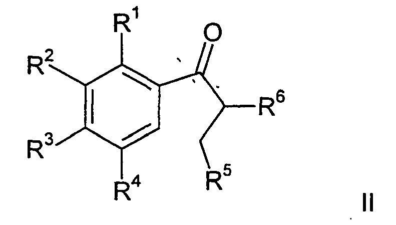 wherein R¹, R², R³, R⁴, R⁵, and R⁶ have the same meaning as defined above,
with a chlorinating agent, followed by reaction with a Friedel-Crafts catalyst.

2. A process according to claim 1, **characterized in that** R¹, R², R³, R⁴, R⁵, and R⁶ independently represent H or a C₁-C₂₀ alkyl group or R¹ and R² or R² and R³ or R³ and R⁴ and/or R⁵ and R⁶ together with the carbon atoms to which they are attached form a saturated or unsaturated 5- or 6-membered ring, said ring optionally being substitued with a C₁-C₄ hydrocarbon group.

3. A process according to claim 1 or 2, **characterized in that** the molar ratio of ketone (II) to chlorinating agent is 1:1 to 1:2.

4. A process according to any one of the preceding claims, **characterized in that** the chlorinating agent is sulfuryl chloride.

5. A process according to any one of the preceding claims, **characterized in that** the molar ratio of Friedel-Crafts catalyst to ketone (II) is 1:1 to 3:1.

6. A process according to any one of the preceding claims, **characterized in that** the Friedel-Crafts catalyst is aluminium chloride or iron (III) chloride.

7. A process according to any one of the preceding claims, **characterized in that** the reaction with a Friedel-Crafts catalyst is carried out in a mixture of a hydrocarbon solvent such as heptane and a halogenated alkane such as dichloromethane.

8. A process for preparing indenes of formula III: 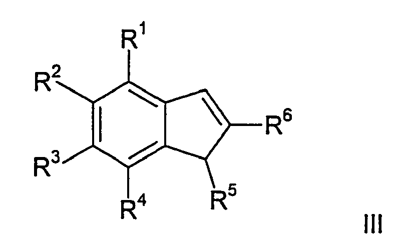 and isomers thereof, wherein R¹, R², R³, R⁴, R⁵ and R⁶ independently represent H or a C₁-C₂₀ hydrocarbon group or R¹ and R² or R² and R³ or R³ and R⁴ and/or R⁵ and R⁶ together with the carbon atoms to which they are attached form a saturated or unsaturated 5- or 6-membered ring, said hydrocarbon group and/or said ring optionally containing one or more hetero atoms, said ring optionally being substituted with a C₁-C₄ hydrocarbon group, **characterized in that** a compound of formula II is converted into a compound of formula I according to the process of any one of the preceding claims, followed by reaction in an inert solvent first with a reducing agent and then with an acid dehydrating agent according to methods known per se.

9. A process according to claim 8, **characterized in that** the reducing agent is selected from the group consisting of sodium borohydride, lithium aluminium hydride, diisobutylaluminium hydride (DIBAL-H), and sodium bis(2-methoxyethoxy)aluminium hydride.

10. A process according to claim 8 or 9, **characterized in that** the acid is selected from the group consisting of sulfuric acid, methanesulfonic acid, p-toluenesulfonic acid, and phosphoric acid.

## Patentansprüche

1. Verfahren zur Herstellung von 1-Indanonen der Formel I: 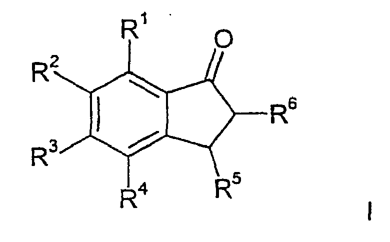 und Isomeren davon, wobei R¹, R², R³, R⁴, R⁵ und R⁶ unabhängig voneinander H oder eine C₁-C₂₀-Kohlenwasserstoffgruppe darstellen oder R¹ und R² oder R² und R³ oder R³ und R⁴ und/oder R⁵ und R⁶ zusammen mit den Kohlenstoffatomen, an die sie gebunden sind, einen gesättigten oder ungesättigten fünf- oder sechsgliedrigen Ring bilden, wobei die Kohlenwasserstoffgruppe und/oder der Ring gegebenenfalls ein oder mehrere Heteroatome enthalten, wobei der Ring gegebenenfalls mit einer C₁-C₄-Kohlenwasserstoffgruppe substituiert ist;
wobei das Verfahren das Umsetzen einer Verbindung der Formel II: 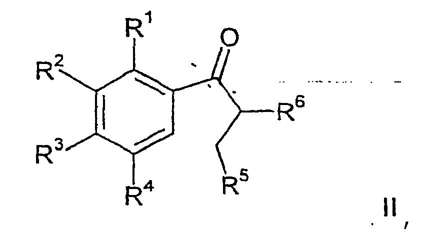 wobei R¹, R², R³, R⁴, R⁵ und R⁶ dasselbe wie oben bedeuten, mit einem Chlorierungsmittel und die anschließende Reaktion mit einem Friedel-Crafts-Katalysator umfasst.

2. Verfahren gemäß Anspruch 1, **dadurch gekennzeichnet, dass** R¹, R², R³, R⁴, R⁵ und R⁶ unabhängig voneinander H oder eine C₁-C₂₀-Alkylgruppe darstellen oder R¹ und R² oder R² und R³ oder R³ und R⁴ und/oder R⁵ und R⁶ zusammen mit den Kohlenstoffatomen, an die sie gebunden sind, einen gesättigten oder ungesättigten fünf- oder sechsgliedrigen Ring bilden, wobei der Ring gegebenenfalls mit einer C₁-C₄-Kohlenwasserstoffgruppe substituiert ist.

3. Verfahren gemäß Anspruch 1 oder 2, **dadurch gekennzeichnet, dass** das Stoffmengenverhältnis von Keton (II) zu Chlorierungsmittel 1:1 bis 1:2 beträgt.

4. Verfahren gemäß einem der vorstehenden Ansprüche, **dadurch gekennzeichnet, dass** es sich bei dem Chlorierungsmittel um Sulfurylchlorid handelt.

5. Verfahren gemäß einem der vorstehenden Ansprüche, **dadurch gekennzeichnet, dass** das Stoffmengenverhältnis von Friedel-Crafts-Katalysator zu Keton (II) 1:1 1 bis 3:1 beträgt.

6. Verfahren gemäß einem der vorstehenden Ansprüche, **dadurch gekennzeichnet, dass** es sich bei dem Friedel-Crafts-Katalysator um Aluminiumchlorid oder Eisen(III)chlorid handelt.

7. Verfahren gemäß einem der vorstehenden Ansprüche, **dadurch gekennzeichnet, dass** die Reaktion mit einem Friedel-Crafts-Katalysator in einem Gemisch aus einem Kohlenwasserstoff-Lösungsmittel, wie Heptan, und einem halogenierten Alkan, wie Dichlormethan, durchgeführt wird.

8. Verfahren zur Herstellung von Indenen der Formel III: 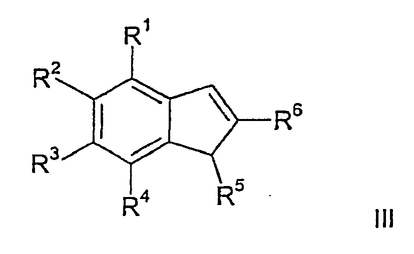 und Isomeren davon, wobei R¹, R², R³, R⁴, R⁵ und R⁶ unabhängig voneinander H oder eine C₁-C₂₀-Kohlenwasserstoffgruppe darstellen oder R¹ und R² oder R² und R³ oder R³ und R⁴ und/oder R⁵ und R⁶ zusammen mit den Kohlenstoffatomen, an die sie gebunden sind, einen gesättigten oder ungesättigten fünf- oder sechsgliedrigen Ring bilden, wobei die Kohlenwasserstoffgruppe und/oder der Ring gegebenenfalls ein oder mehrere Heteroatome enthalten, wobei der Ring gegebenenfalls mit einer C₁-C₄-Kohlenwasserstoffgruppe substituiert ist, **dadurch gekennzeichnet, dass** eine Verbindung der Formel II nach einem Verfahren gemäß einem der vorstehenden Ansprüche in eine Verbindung der Formel I umgewandelt wird, gefolgt von einer Reaktion in einem inerten Lösungsmittel, zuerst mit einem Reduktionsmittel und dann mit einem Säuredehydratisierungsmittel nach an sich bekannten Verfahren.

9. Verfahren gemäß Anspruch 8, **dadurch gekennzeichnet, dass** das Reduktionsmittel aus der Gruppe ausgewählt ist, die aus Natriumborhydrid, Lithiumaluminiumhydrid, Diisobutylaluminiumhydrid (DIBAL-H) und Natriumbis(2-methoxyethoxy)aluminiumhydrid besteht.

10. Verfahren gemäß Anspruch 8 oder 9, **dadurch gekennzeichnet, dass** die Säure aus der Gruppe ausgewählt ist, die aus Schwefelsäure, Methansulfonsäure, p-Toluolsulfonsäure und Phosphorsäure besteht.

## Revendications

1. Procédé de préparation de 1-indanones de formule I : 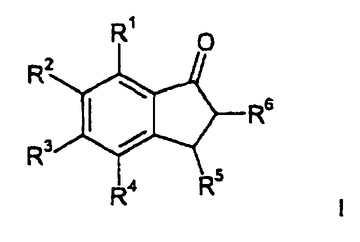 et des isomères de ceux-ci, dans laquelle R¹, R², R³, R⁴, R⁵, et R⁶ représentent indépendamment H ou un groupe hydrocarbure en C₁-C₂₀ ou R¹ et R² ou R² et R³ ou R³ et R⁴ et/ou R⁵ et R⁶, conjointement avec les atomes de carbone auxquels ils sont liés, forment un cycle à 5 ou 6 chaînons saturé ou insaturé, ledit groupe hydrocarbure et/ou ledit cycle contenant facultativement un hétéroatome ou plus, ledit cycle étant facultativement substitué par un groupe hydrocarbure en C₁-C₄,
ledit procédé comprenant la réaction d'un composé de formule II : 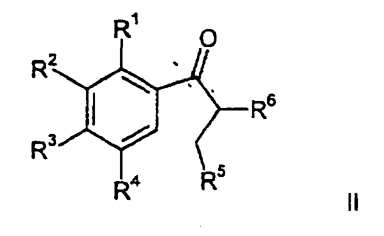 dans laquelle R¹,R², R³, R⁴, R⁵, et R⁶ ont la même signification que celle définie ci-dessus,
avec un agent de chloration, suivie par la réaction avec un catalyseur de Friedel-Crafts.

2. Procédé selon la revendication 1, **caractérisé en ce que** R¹, R², R³, R⁴, R⁵, et R⁶ représentent indépendamment H ou un groupe alkyle en C₁-C₂₀ ou R¹ et R² ou R² et R³ ou R³ et R⁴ et/ou R⁵ et R⁶, conjointement avec les atomes de carbone auxquels ils sont liés, forment un cycle à 5 ou 6 chaînons saturé ou insaturé, ledit cycle étant facultativement substitué par un groupe hydrocarbure en C₁-C₄.

3. Procédé selon la revendication 1 ou 2, **caractérisé en ce que** le rapport molaire de la cétone (II) sur l'agent de chloration est de 1/1 à 1/2.

4. Procédé selon l'une quelconque des revendications précédentes, **caractérisé en ce que** l'agent de chloration est le chlorure de sulfuryle.

5. Procédé selon l'une quelconque des revendications précédentes, **caractérisé en ce que** le rapport molaire du catalyseur de Friedel-Crafts sur la cétone (II) est de 1/1 à 3/1.

6. Procédé selon l'une quelconque des revendications précédentes, **caractérisé en ce que** le catalyseur de Friedel-Crafts est le chlorure d'aluminium ou le chlorure de fer (III).

7. Procédé selon l'une quelconque des revendications précédentes, **caractérisé en ce que** la réaction avec un catalyseur de Friedel-Crafts est réalisée dans un mélange d'un solvant hydrocarboné tel que l'heptane et d'un alcane halogéné tel que le dichlorométhane.

8. Procédé de préparation d'indènes de formule III : 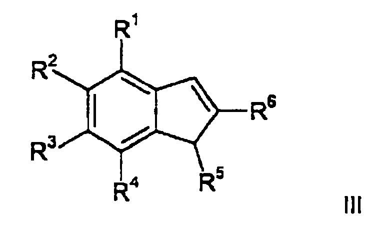 et des isomères de ceux-ci, dans laquelle R¹, R², R³, R⁴, R⁵ et R⁶ représentent indépendamment H ou un groupe hydrocarbure en C₁-C₂₀ ou R¹ et R² ou R² et R³ ou R³ et R⁴ et/ou R⁵ et R⁶, conjointement avec les atomes de carbone auxquels ils sont liés, forment un cycle à 5 ou 6 chaînons saturé ou insaturé, ledit groupe hydrocarbure et/ou ledit cycle contenant facultativement un hétéroatome ou plus, ledit cycle étant facultativement substitué par un groupe hydrocarbure en C₁-C₄, **caractérisé en ce qu'**un composé de formule II est converti en un composé de formule I selon le procédé selon l'une quelconque des revendications précédentes, suivi par la réaction d'un solvant inerte d'abord avec un agent de réduction puis avec un agent déshydratant acide selon des procédés connus en soi.

9. Procédé selon la revendication 8, **caractérisé en ce que** l'agent de réduction est choisi dans le groupe constitué par le borohydrure de sodium, l'hydrure de lithium aluminium, l'hydrure de diisobutylaluminium (DIBAL-H), et l'hydrure de sodium bis(2-méthoxyéthoxy)aluminium.

10. Procédé selon la revendication 8 ou 9, **caractérisé en ce que** l'acide est choisi dans le groupe constitué par l'acide sulfurique, l'acide méthanesulfonique, l'acide p-toluènesulfonique, et l'acide phosphorique.
